# EUROPEAN PATENT APPLICATION

(11) **EP 1 023 897 A2**
(43) Date of publication of application: **02.08.2000**
(21) Application number: 00101431.5
(22) Date of filing: 25.01.2000
(51) Int. Cl.: A61K 31/375, A61K 33/30, A61P 17/10

(54) **Dermal agent**

(30) Priority: 26.01.1999 JP 1747899
(71) Applicant: SHOWA DENKO KABUSHIKI KAISHA, Minato-ku, Tokyo (JP)
(72) Inventor: Masatsuji, Eiko, c/o Central Res. Lab. Showa Denko, Chiba-shi, Chiba 267-0056 (JP); Tsuzuki, Toshi, c/o Central Res. Lab. Showa Denko, Chiba-shi, Chiba 267-0056 (JP); Ito, Shinobu, c/o Showa Denko K.K., Tokyo 105-8518 (JP); Ogata, Eiji, Showa Denko K.K., Tokyo 105-8518 (JP)
(74) Representative: Strehl Schübel-Hopf & Partner

(57) **Abstract**

A dermal agent for preventing or treating acne, comprising an ascorbic acid derivative which liberates in vivo ascorbic acid, and a zinc salt or comprising a zinc salt of the ascorbic acid-2-phosphate, and a composition comprising tretinoin and an ascorbic acid derivative or a salt thereof, relieving the irritation of tretinoin by using the dermal agent and tretinoin in combination.

## Description

### FIELD OF THE INVENTION

The present invention relates to a dermal agent comprising an ascorbic acid derivative which is degraded in vivo to liberate ascorbic acid, and a zinc salt, such as a dermal agent having an effect of preventing or treating comedones, an antibactrial dermal agent, a dermal agent having an inhibitory effect on growth of *Propionibacterium* and a dermal agent having an inhibitory effect on growth of *Staphylococcus*. The present invention also relates to a dermal agent where the irritation caused by tretinoin is eliminated by using the above-described dermal agent in combination with tretinoin.

### BACKGROUND OF THE INVENTION

Acne (acne vulgaris) is a chronic skin disease having a high incidence mainly at puberty. A large number of physiological or microbiological factors participate in the pathogenesis or pathophygiology. The disease is a result of complicated interaction of these factors. A main causative factor of incipient acne is excess secretion of lipid from pilosebaceous unit.

Androgen as a sex hormone is greatly responsible for this excess secretion. When hormone balance is altered in vivo, excess secretion of lipid is induced and the resulting obstruction of hair follicles and subsequent proliferation of microorganisms give rise to lesions.

In the next stage of acne, bacterial colonization in the inside or periphery of sebaceous glands is the causative factor. In particular, *Propionibacterium acnes* plays an important role as a pathogenic bacterium. This bacterium is an obligately anaerobic microorganism and occurs ubiquitously on the human skin. The growth thereof aggressively takes place in hair follicles changed to be anaerobic as a result of obstruction by lipid. The bacterium when growing secretes lipase, hyaluronidase and protease. Lipase hydrolyzes lipid to liberate fatty acid causing high skin irritation. As a result, inflammation occurs. Hyaluronidase and protease each invades the inflamed skin, dissolves the texture and thereby enlarges the degree of inflammation. These enzymes have very grave relations to the pathophygiology of comedones, nevertheless, inhibitors against these enzymes are not used positively. Among acne treating agents currently used, a few have an effect of inhibiting lipase. However, a test by the present inventors revealed that the effect is very low and cannot be admitted to be a principal effect in prevention or treatment.

*Staphylococcus aureus* is another microorganism participating together with *Propionibacterium acnes* in the acne pathophysiology. This microorganism is understood to infect the portion of the skin where the initial inflammation occurs, and secondarily to intensify and enlarge the inflammation to aggravate general disease conditions.

As the antibacterial treating agent against these peccant microorganisms, antibiotics such as clindamycin and erythromycin, and tretinoin predominate in Europe and the U.S. In Japan, medicaments containing antibacterial ingredients such as a sulfur agent are being used. However, antibiotics have the inevitable problem that resistant microorganisms appear during use over a long period of time, despite their excellent antibacterial activity. Tretinoin has a problem in that frequent use thereof brings out toxicity or the preparation containing tretinoin has strong irritation on skin and is difficult to use. Furthermore, sulfur agent and the like cannot promise at present sufficiently high antibiotic activity.

In recent years, it is pointed out that active oxygen inside or on the skin is the etiology of comedones. Particularly, it has been reported that production of hydroxyl radicals under irradiation of ultraviolet rays is conspicuously increased by the presence of coproporphyrin secreted from *Propionibacterium*. As a comedolytic ingredient having a purpose of eliminating active oxygen, particularly hydroxyl radicals, hydroquinone or natural product-derived polyphenols or the like has been proposed. However, these ingredients are highly irritating to the skin and it may be duly presumed that existing inflammation is further aggravated.

After inflammation is once settled, the skin structural texture is destroyed by the action of protease or collagenase secreted in the skin. As a result, the skin is depressed and scarring with many uneven pockmarks occurs. This usually requires a very long time to restore sound skin.

An ingredient of promoting reproduction of collagen after damage is considered to have very high effect on improvement or prevention of such sequelae. However, none of the ingredients for acne treating agent known and used at present has such effects.

In addition to the depression of skin, melanotic pigmentation after inflammation is a serious problem and the self confidence of sufferers is greatly undermined after healing. In a similar manner to the promotion of collagen synthesis, an ingredient for preventing pigmentation of skin is not positively used in acne treating agents. Ellagic acid, kojic acid and the like are used as a whitening cosmetic ingredient for removing skin pigmentation. However, these are used ultimately for the cosmetic purpose.

As such, preventive or treating medical products heretofore known fail in effecting complicated and diversified pathophysiologies in all stages from crisis through healing. When healing of comedones is required, it must be considered that those pathophysiologies all are simultaneously proceeding side by side on the skin to which the medicament is applied. The effect required cannot be satisfactorily attained by any means if one of, for example, a hormone agent, an antibiotic, an antiinflammatory, an antioxidant or a whitening agent is used alone. Depending on the case, these may be applied in combination, however, in view of physical properties and physiological properties of the various medicaments, a preparation using a mixture thereof is difficult to formulate in many cases.

Therefore, an agent for preventing or treating acne, having all of an antibacterial property, an enzyme inhibitory activity, an antioxidant property, a collagen reproduction promoting action and a pigmentation preventing action, ensuring high safety in the administration over a long period of time, and being pharmaceutically easy to handle, is keenly demanded.

### SUMMARY OF THE INVENTION

An object of the present invention is to provide a dermal agent for preventing or treating acne, having various functions of anti-bacterial activity, inhibition of lipase and hyaluronidase, oxidation inhibition, induction of synthesis of collagen and prevention of melanotic pigmentation, thereby exhibiting the effect of improving general symptoms of acne, being highly safe and almost free of irritation even in application over a long period of time, and being pharmaceutically easy to handle.

Under these circumstances, the present inventors have made extensive investigations. As a result, it has been found that a mixture of an ascorbic acid derivative or a salt thereof with a zinc salt compound, particularly a mixture of an ascorbic acid-2-phosphate or a salt thereof with a zinc salt compound, and an ascorbic acid-2-phosphate zinc salt can maintain the above-described functions in good balance and in turn exhibit excellent acne preventing effect and also superior acne treating effect including removal of scars remaining after healing. The present invention has been accomplished based on this finding.

More specifically, the above-described object can be obtained by the present invention comprising the following embodiments:
(1) a dermal agent for preventing or treating comedones, comprising an ascorbic acid derivative which liberates ascorbic acid in vivo, or a salt thereof and a zinc salt compound or comprising a zinc salt of the ascorbic acid derivative;
(2) an antibacterial dermal agent comprising an ascorbic acid derivative which liberates ascorbic acid in vivo, or a salt thereof and a zinc salt compound or comprising a zinc salt of the ascorbic acid derivative;
(3) a dermal agent having an inhibitory effect on growth of *Propionibacterium*, comprising an ascorbic acid derivative which liberates ascorbic acid in vivo, or a salt thereof and a zinc salt compound or comprising a zinc salt of the ascorbic acid derivative;
(4) a dermal agent having an inhibitory effect on growth of *Staphylococcus* comprising an ascorbic acid derivative which liberates ascorbic acid in vivo, or a salt thereof and a zinc salt compound or comprising a zinc salt of the ascorbic acid derivative;
(5) a denial agent comprising an ascorbic acid derivative which liberates ascorbic acid in vivo, or a salt thereof and a zinc salt compound or comprising a zinc salt of the ascorbic acid derivative, the denial agent having an inhibitory activity against lipase derived from microorganisms;
(6) a denial agent comprising an ascorbic acid derivative which liberates ascorbic acid in vivo, or a salt thereof and a zinc salt compound or comprising a zinc salt of the ascorbic acid derivative, the dermal agent having an inhibitory activity against hyaluronidase derived from microorganisms;
(7) the denial agent as described in any one of (1) to (6) above, wherein the ascorbic acid derivative which liberates ascorbic acid in vivo is a compound represented by the following formula (1): 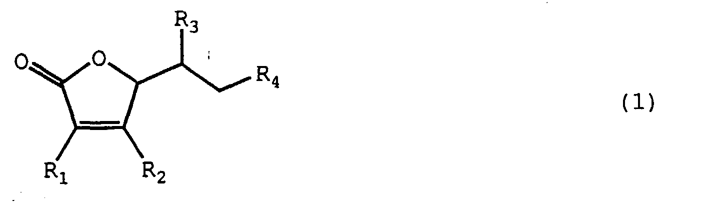 wherein R¹ and R² each represents a hydroxyl group, a phosphoric acid group, a pyrophosphoric acid group, a triphosphoric acid group, a polyphosphoric acid group, an O-glucosyl group, a sulfuric acid group, or an acyloxy group which may contain a branched or unsaturated bond, R³ and R⁴ each represents a hydroxyl group, a phosphoric acid group, a pyrophosphoric acid group, a triphosphoric acid group, a polyphosphoric acid group, an O-glucosyl group, a sulfuric acid group, an acyloxy group which may contain a branched or unsaturated bond, an alkyloxy group which may contain a branched or unsaturated bond, or a hydroxyalkyloxy group, and R³ and R⁴ may be bonded as an acetal or ketal to the same carbon atom through an oxygen atom, provided that R¹ and R² are not a hydroxyl group at the same time;
(8) the dermal agent as described in any one of (1) to (6) above, wherein the salt of an ascorbic acid derivative which liberates ascorbic acid in vivo is a salt of ascorbic acid-2-phosphate represented by the following formula (2): 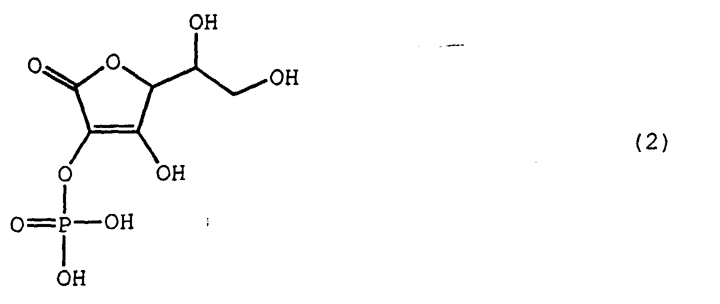
(9) the dermal agent as described in any one of (1) to (6) above, wherein the zinc salt of an ascorbic acid derivative which liberates ascorbic acid in vivo is an ascorbic acid-2-phosphate zinc salt represented by the following formula (3): 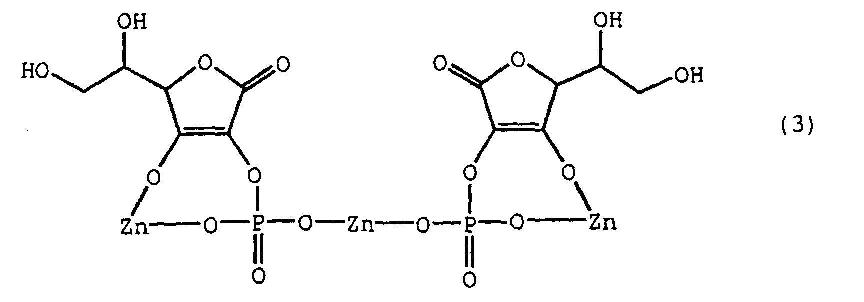
(10) the dermal agent as described in any one of (1) to (6) above, wherein the ascorbic acid derivative which liberates ascorbic acid in vivo is ascorbic acid-2-O-glucoside;
(11) a composition comprising tretinoin and an ascorbic acid derivative or a salt thereof, wherein irritation of tretinoin is relieved by using the dermal agent described in any one of (1) to (10) above in combination with tretinoin; and
(12)a method for relieving the irritation of tretinoin, comprising using the dermal agent described in any one of (1) to (10) above in combination with tretinoin.

### DESCRIPTION OF THE PREFERRED EMBODIMENTS

The denial agent of the present invention applied to skin provides a condition that an ascorbic acid derivative and zinc ion are dissolved and are present together. In this state, the ascorbic acid derivative and zinc ion are connected to assume a chelate form and thereby produce a complex. As a result, a high antibacterial effect against *Propionibacterium* and *Staphylococcus* and a high antienzymatic activity against lipase and hyaluronidase can be achieved.

Several kinds of ascorbic acid derivatives, particularly ascorbic acid-2-phosphate and ascorbic acid-2-glucoside, are already known to liberate ascorbic acid in vivo to thereby increase intracellular ascorbic acid concentration, whereby high effects superior to those in the administration of ascorbic acid itself can be obtained, such as resistance to intradermal or intracellular oxidation, activity of inhibiting decomposition or promoting reproduction of collagen, and capability of preventing or removing pigmentation of the skin (see, Yamane et al., Fragrance Journal, Vol. 25, No. 3, pp. 7-19 (1997): Ichihashi et al., ibid., pp. 29-33; Kobayashi et al., ibid., pp. 34-40; Sugimoto et al., ibid., pp. 41-54; Sakamoto et al., ibid., pp. 62-70; and Shitomi et al., ibid., pp. 80-85).

However, none of the known ascorbic acid derivatives and dermal agents containing the ascorbic acid derivative has an antibacterial effect and an antienzymatic activity as high as the dermal agent of the present invention. It is now further required to develop an ascorbic acid derivative having these effects and at the same time, conventionally known effects and to synergistically exhibit excellent resistance to acne, or a salt thereof, and also to develop a dermal agent containing the ascorbic acid derivative or a salt thereof.

The ascorbic acid derivative or a salt thereof for use in the present invention may be any such compound as long as it can undergo in vivo enzymatic or nonenzymatic decomposition and thereby liberate ascorbic acid, and at the same time can form a complex with zinc ion.

Examples of suitable ascorbic acid derivatives and salts thereof having these properties include ascorbic acid-2-phosphate, ascorbic acid-2-pyrophosphate, ascorbic acid-2-triphosphate, ascorbic acid-2-polyphosphate, ascorbic acid-2,3-diphosphate, ascorbic acid-2,6-diphosphate, ascorbic acid-2-sulfate, ascorbic acid-6-palmitate, ascorbic acid-2,6-palmitate, ascorbic acid-2-glucoside, ascorbic acid-2-O-glucoside-6-palmitate, ascorbic acid-5,6-benzilidene, ascorbic acid-5,6-propylidene, and metal salts, ammonium salts and alkyl- or hydroxyalkyl-substituted ammonium salts thereof.

Among these ascorbic acid derivatives, ascorbic acid-2-phosphate and salts thereof such as ascorbic acid-2-phosphate magnesium salt and ascorbic acid-2-phosphate sodium salt are preferred in view of effect and efficacy. The ascorbic acid-2-phosphate is transported into a living body at a high rate as compared with other known ascorbic acid derivatives and liberates ascorbic acid in vivo at a high rate (see, Yamane et al., Fragrance Journal, Vol. 25, No. 3, pp. 7-19 (1997)).

The salts can be produced, for example, by the method described in JP-A-44-31237 (the term "JP-A" as used herein means an "unexamined published Japanese patent application"). Commercially available products may also be used.

The zinc salt compound for use in the present invention may be any such salt as long as the toxicity is not seriously high. Examples thereof include inorganic salts such as zinc chloride, zinc ammonium chloride, zinc carbonate, zinc nitrate, zinc sulfate, zinc sulfide, zinc borate, zinc phosphate and zinc pyrophosphate, and organic salts such as zinc acetate, zinc benzoate, zinc lactate, zinc citrate, zinc oxalate and zinc tartrate. Among these, zinc chloride and zinc acetate are preferred from the standpoint of water solubility and toxicity.

Preferred examples of the mixture of an ascorbic acid derivative or a salt and a zinc salt compound include a mixture of a salt other than ascorbic acid-2-phosphate zinc salt with a zinc salt compound. Preferred examples of the zinc salt of an ascorbic acid derivative include ascorbic acid-2-phosphate zinc salt. These may also be used in combination.

The ascorbic acid-2-phosphate zinc salt may be obtained, for example, by the method described in Japanese Patent Application No. 9-153972. More specifically, according to this method, a commercially available ascorbic acid-2-phosphate salt such as ascorbic acid-2-phosphate magnesium salt, and a zinc salt such as zinc chloride are used as starting materials and the metal ion present in the ascorbic acid-2-phosphate salt is displaced by the zinc ion using ion exchange chromatography or the like. The thus-obtained ascorbic acid-2-phosphate zinc salt does not completely dissociate in crystal or aqueous solution but depending on the conditions, partly or entirely forms an ascorbic acid-2-phosphate zinc complex having a structure represented by formula (2) herein.

The dermal agent containing an ascorbic acid-2-phosphate zinc salt according to the present invention can be obtained, for example, as follows.

An ascorbic acid-2-phosphate zinc salt powder prepared by the above-described method is used as it is or after dissolving it in an aqueous solution or other pharmaceutically acceptable solution. The solubility may be low depending on the kind of solution and in such a case, the powder may be, if desired, dispersed by adding a dispersant or the like or solubilized by adding a solubilizing agent or the like.

The dermal agent containing ascorbic acid-2 -phosphate or a salt thereof and a zinc salt compound according to the present invention may be obtained, for example, as follows.

An ascorbic acid-2-phosphate salt such as ascorbic acid-2-phosphate sodium salt or ascorbic acid-2-phosphate magnesium salt, and a zinc salt such as zinc chloride or zinc acetate, are mixed at an arbitrary ratio as crystals or a powder intact or in water or other solvent, thereby obtaining the denial agent. In the case where these are mixed in an aqueous solution or a water-containing solution, the ascorbic acid-2-phosphate salt dissociates and depending on the conditions, partly or entirely combines with zinc ion to form an ascorbic acid-2-phosphate zinc complex, presenting the same state as in the case of the ascorbic acid-2-phosphate zinc salt being dissolved in water.

In the case where these materials are mixed as crystals or a powder, the same state may also be achieved as a result of the mixture being dissolved in water or formed into an aqueous solution on the skin or in vivo.

In the dermal agent of the present invention, the ascorbic acid-2-phosphate zinc salt or a mixture compound of the ascorbic acid-2-phosphate or a salt thereof with a zinc salt may be the sole active ingredient or one of a number of active ingredients.

In either case, the amount of ascorbic acid-2-phosphate zinc salt in a preparation naturally varies depending on the objective patient, symptom or drug form. However, it is usually from 0.01 to 90% by weight and from the standpoint of effect and facility as a preparation, preferably from 0.05 to 20% by weight.

The content in total of the ascorbic acid-2-phosphate or a salt thereof and the zinc salt compound in a preparation is similarly from 0.01 to 90% by weight and from the standpoint of effect and facility as a preparation, preferably from 0.02 to 30% by weight. In this case, the ascorbic acid-2-phosphate or a salt thereof and the zinc salt compound may be mixed at any ratio, however, in view of the effect, the molar ratio between the ascorbic acid-2-phosphate and the zinc salt compound is preferably 1:0.1 to 10, more preferably close to 1:1.5.

In addition to the above-described ingredients, the dermal agent of the present invention may be used in a medicament together with other active ingredients by optionally adding a comedolytic agent, an antiandrogen, an antimicrobial, an antiinflammatory, an antioxidant, a radical scavenger, a whitening agent and the like conventionally used as an active ingredient.

Examples of antiandrogen active ingredients which can be used in combination include cyproterone acetate, spironolactone, estrogen and glucocorticoid. Examples of antimicrobial active ingredients which can be used in combination include antibiotics such as erythromycin, clindamycin, gentamycin, penicillin, chloramphenicol and tetracycline, and antimicrobial ingredients such as benzoyl peroxide, nadifloxacin, ethanol, benzalkonium chloride, sulfur, parahydroxybenzoate esters, salicylic acid, hinokitiol, triclosan and homosulfamine. Examples of antiinflammatory active ingredients which can be used in combination include ibuprofen PICONOL, glycyrrhizin, camphor and indomethacin.

Examples of comedolytic agent active ingredients which can be used in combination include tretinoin, resorcin, isopropylmethylphenol, tocopherol and ascorbic acid.

Examples of whitening agent active ingredients which can be used in combination include placenta extract, kojic acid, ellagic acid, arbutin and tranexamic acid ester.

Other than these, antimicrobial, antioxidant and antiinflammatory ingredients may also be used in combination, such as chamomile extract, Sasa Albo-marginata extract, rose extract, balm mint extract, gentian extract, glycyrrhiza extract, jojoba extract, rosemary extract, sage extract, wild thyme extract, lavender extract, paeonia extract, ginseng extract, aloe extract, glycine extract, soy extract, perilla extract, mugwort extract, tumeric extract, Japanese cypress leaf extract, Japanese cypress extract, Rhei Rhizoma extract, phellodendron bark extract, Japanese coptis extract, ginkgo extract, mulberry bark extract, tea extract, grape peel extract, Eleutherococcus senticosus extract, gynostemma pentaphyllum extract and various seaweed extracts.

These active ingredients used in combination each is added in an amount of from 0.01 to 50% by weight though the amount added can vary depending on the kind and use thereof.

The dermal agent of the present invention may be used after forming it together with a preparation carrier into any preparation form suitable for the use by a known preparation techniques. Examples of forms include poultices, patches, oily ointments, aqueous ointments, hard ointments, liniments, gels, creams, cosmetic lotions, lotions, emulsions, face lotions, packs, plasters, soaps, face washes, body soaps, hair treatments and rinses.

In the formulation of these preparations, ingredients commonly used in dermal agents in general may be used. Examples thereof include surfactants, oils, alcohols, moisture retentates, thickeners, antiseptics, antioxidants, chelating agents, pH conditioners, perfumes, dyes, ultraviolet absorbents, ultraviolet scattering agents and amino acids.

Examples of surfactants which can be used include nonionic surfactants such as glycerin monostearate, polyglycerin monostearate, sorbitan monooleate, polyethylene glycol monostearate, polyoxyethylene monooleate, polyoxyethylene cetyl ether, polyoxyethylenated sterol, polyoxyethylenated lanolin and polyoxyethylene hydrogenated castor oil, anionic surfactants such as sodium stearate, potassium palmitate, sodium cetylsulfate, sodium lauryl sulfate, triethanolamine palmitate, sodium polyoxyethylenelauryl phosphate, sodium acylglutamate and SURFACTIN, cationic surfactants such as stearyldimethylbenzylammonium chloride and stearyltrimethylammonium chloride, and amphoteric surfactants such as alkylamino-ethylglycine chloride and lecithin.

Examples of oils which can be used include vegetable fats and oils such as castor oil, olive oil, cacao oil, tsubaki oil, coconut oil, Japan wax, jojoba oil, grape seed oil and avocado oil, animal oils and fats such as mink oil and egg yolk oil, waxes such as beeswax, spermaceti, lanolin, carnauba wax and candelilla wax, hydrocarbons such as liquid paraffin, squalane, microcrystalline wax, ceresin oil, paraffin wax and petrolatum, natural and synthetic fatty acids such as lauric acid, myristic acid, stearic acid, oleic acid, isostearic acid and behenic acid, natural and synthetic higher alcohols such as cetanol, stearyl alcohol, hexyldecanol, octyldodecanol and lauryl alcohol, and esters such as isopropyl myristate, isopropyl palmitate, isopropyl adipate, octyldodecyl myristate, octyldodecyl oleate and cholesterol oleate.

Examples of moisture retentates which can be used include polyhydric alcohols such as glycerin, propylene glycol, 1,3-butylene glycol, sorbitol, polyglycerin, polyethylene glycol and dipropylene glycol, NMF ingredients such as sodium lactate, and water-soluble polymers such as hyaluronic acid, collagen, monopolysaccharide and chondroitin sulfuric acid.

Examples of thickeners which can be used include natural polymers such as sodium alginate, xanthan gum, aluminum silicate, quince seed extract, tragacanth gum and starch, and semisynthetic polymers such as methyl cellulose, hydroxyethyl cellulose, carboxymethyl cellulose, cationized starch and cationized cellulose.

Examples of chelating agents which can be used include edetate, pyrophosphate, hexametaphosphate, citric acid, tartaric acid and gluconic acid. Examples of pH conditioners which can be used include sodium hydroxide, triethanolamine, hydrochloric acid, citric acid and salts thereof, boric acid, borax, potassium hydrogenphosphate and sodium hydrogenphosphate.

Examples of ultraviolet absorbents which can be used include p-amino acid type, salicylic acid type, benzofuran type, coumarin type and azole type compounds, such as 2-hydroxy-4-methoxybenzophenone, octyldimethyl p-aminobenzoate and ethylhexyl p-methoxycinnamate.

Furthermore, an ultraviolet scattering agent such as titanium oxide, kaolin or talc may also be added.

Use of the composition containing tretinoin and an ascorbic acid derivative or a salt thereof according to the present invention is not limited only to comedones but the composition may be applied to all uses to which tretinoin can be applied. The use includes effects on dermatitis or promotion of hair growth.

For example, the ascorbic acid derivative can be used also in the mixture formulation of a hair growing ingredient such as minoxidil with tretinoin.

In Europe and U.S., tretinoin is usually used in an amount of from 0.05 to 0.1% by weight, however, depending on the patient, irritating symptom such as rash and eczema may appear on the skin even with 0.01% by weight of tretinoin. In such a case, it is most effective to use from 0.1 to 0.001% by weight of tretinoin in combination with from 0.1 to 10 wt% by weight of the dermal agent of the present invention comprising an ascorbic acid composition which liberates in vivo ascorbic acid, or a salt and a zinc salt compound or comprising a zinc salt of an ascorbic acid derivative which liberates in vivo ascorbic acid.

The dermal agent of the present invention can be used as a poultice by holding the dermal agent in a hydrophilic polymer. In particular, a poultice obtained by holding the dermal agent in a hydrophilic polymer having the property of sustained releasability is preferred.

The hydrophilic polymer is not particularly limited as long as it has no problem in view of skin irritation, however, hydrophilic polymers commonly used for poultices are preferably used. Examples thereof include acrylic acid (salt) polymers, N-vinylcarboxylic acid amide polymers, polyvinyl alcohols and acrylamide polymers.

Among these, N-vinylcarboxylic acid polymers such as N-vinylacetamide polymers are preferred.

Such polymers may be obtained, for example, by copolymerizing from 60 to 95 mass% of N-vinylacetamide and from 5 to 40 mass% of a copolymerizable compound having an ethylenic double bond (with the total of all monomers being 100 mass%) in water in the presence of a polymerization initiator in a monomer concentration of from 10 to 40 mass%.

### EXAMPLES

The present invention is described in greater detail below by referring to the following Examples. However, the present invention should not be construed as being limited by these Examples. Unless otherwise indicated herein, all parts, percents, ratios and the like are by weight.

### Example 1:

### (Synthesis of Ascorbic Acid-2-Phosphate Zinc Salt)

Ascorbic acid-2-phosphate zinc salt was synthesized according to the method described in Japanese Patent Application No. 9-153972.

More specifically, cationic exchange resin Diaion SK1B (produced by Mitsubishi Chemical Corporation) was packed in a glass-made column having a diameter of 5 cm to a height of 20 cm, and 1,500 ml of 1M zinc sulfate and 500 ml of water were added in this order at a flow rate of 10 ml/min to convert the resin to a zinc type resin.

Thereto, 500 ml of a 10% aqueous solution of L-ascorbyl-2-phosphate magnesium salt (L-Ascorbyl PM, produced by Showa Denko K.K.) and 500 ml of water were added in this order at a flow rate of 10 ml/min, the eluent was collected and the entire amount was freeze-dried to obtain 52 g of ascorbic acid-2-phosphate zinc salt powder.

1 mg of the powder obtained was dissolved in 10 ml of water, 0.01 ml of the resulting solution was injected into a high performance liquid chromatograph equipped with an ion exchange column Shodex IEC DEAE-825 (produced by Showa Denko K.K.) to elute the solution through an aqueous ammonium acetate solution using the gradient from 10 mM to 1M and the eluent was analyzed by the detection in the ultraviolet region of 265 nm. As a result, the content of ascorbic acid-2-phosphate in the sample powder was 58.6%.

A small amount of the ascorbic acid-2-phosphate zinc salt powder prepared above was sampled and subjected to elemental analysis by Model MT-3 Elemental Analyzer (manufactured by Yanagimoto Seisakusho K.K.) using tungsten trioxide as a combustion improver. The elemental weight composition was C: 16.7%, H: 3.5%, O: 50.1%.

Subsequently, a small amount of the ascorbic acid-2-phosphate zinc salt powder prepared above was sampled and 31 Pnmr was measured by Model AMX400 nmr Analyzer (manufactured by Bruker K.K.) to calculate the P content. Then, the P content was 7.2%.

Thereafter, a small amount of the ascorbic acid-2-phosphate zinc salt powder prepared above was sampled and the metal ion amount was measured by an ICP emission method and found that Zn content was 22.5% and Mg content was 0.1% or less.

Furthermore, a small amount of the ascorbic acid-2-phosphate zinc salt powder prepared above was sampled and the water content was measured by Model MCICA-05 Karl Fischer's Moisture Meter (manufactured by Mitsubishi Chemical Corporation). Then, 4.5 molecule of water was detected per molecule of ascorbic acid-2-phosphate.

From these results, the chemical formula of the ascorbic acid-2-phosphate zinc salt obtained was determined as AP₂Zn₃·9H₂O (AP represents ascorbic acid-2-phosphate ion).

A small amount of this ascorbic acid-2-phosphate zinc salt powder was sampled and subjected to FAB-MS analysis using a Model JEOLSX102A Mass Spectrometer (manufactured by Nippon Bunko K.K.). As a result, a pseudo molecule ion peak of m/z=703 was detected and the presence of a complex structure represented by formula (2) was confirmed.

This powder was used in the following tests as the standard ascorbic acid-2-phosphate zinc salt (hereinafter referred to as "APZ").

### Example 2:

### (Production Process of Ascorbic Acid-2-Phosphate and Zinc Salt Mixture)

10.0 g of L-ascorbic acid-2-phosphate magnesium salt (L-Ascorbic Acid PM, produced by Showa Denko K.K., hereinafter referred to as "APM") and 8.0 g of zinc chloride (produced by Sigma) were placed in a mortar and thoroughly pulverized and mixed.

This powder was used in the following tests as the standard mixture of ascorbic acid-2-phosphate and zinc salt (hereinafter referred to as "AP+Zn").

### Example 3:

### (Production Process of Ascorbic Acid-2-O-Glucoside and Zinc Salt Mixture)

10.0 g of L-ascorbic acid-2-O-glucoside (produced by Hayashibara Seibutsu Kagaku Kenkyusho, hereinafter referred to as "AG") and 8.0 g of zinc chloride (produced by Sigma) were placed in a mortar and thoroughly pulverized and mixed.

This powder was used in the following tests as the standard mixture of ascorbic acid-2-glucoside and zinc salt (hereinafter referred to as "AG+Zn").

### Example 4:

### (Antibacterial Action against Propionibacterium)

By the measurement of minimum inhibitory concentration (MIC), the ascorbic acid-2-phosphate zinc salt, the ascorbic acid-2-phosphate and zinc salt mixture and the ascorbic acid-2-glucoside and zinc salt mixture of the present invention were determined on their antibacterial action against *Propionibacterium*.

A platinum-loopful of cells of fresh *Propionibacterium acnes* JCM6425 strain were inoculated in a test tube containing 5 ml of GAM broth medium (produced by Nissui K.K.) previously sterilized and deaerated. Thereto, APZ, AP+Zn and AG+Zn prepared as in Examples 1 to 3, APM and AG, each diluted in a common ratio of 2 to have a concentration of from 10 to 0.078 mg/ml, were added and cultured at 35°C for 72 hours under anaerobic conditions. After completion of cultivation, the test tube was thoroughly shaken and the turbidity of the culture solution was measured. The minimum concentration when the turbidity was not increased by cultivation was used as the minimum inhibitory concentration (MIC). The results obtained are shown in Table 1 below. APZ, AP+Zn and AG+Zn were verified to have antibacterial activity.

**Table 1**

| Sample | MIC (mg/ml) |
|---|---|
| APZ | 0.313 |
| AP+Zn | 0.625 |
| AG+Zn | 1.25 |
| APM | >10 |
| AG | >10 |

### Example 5:

### (Antibacterial Action against Staphylococcus)

By measurement of minimum inhibitory concentration (MIC), the antibacterial action against *Staphylococcus* of the ascorbic acid-2-phosphate zinc salt, the ascorbic acid-2-phosphate and zinc salt mixture and the ascorbic acid-2-glucoside and zinc salt mixture of the present invention was determined.

A platinum-loopful of cells of fresh *Staphylococcus aureus* IFO12732 strain were inoculated in a test tube containing 5 ml of nutrient broth medium (produced by Difco) previously sterilized and deaerated. Thereto, APZ, AP+Zn and AG+Zn prepared in Examples 1 to 3, APM and AG, each diluted in a common ratio of 2 to have a concentration of from 10 to 0.078 mg/ml, were added and cultured under shaking at 35°C for 48 hours. After the completion of cultivation, the turbidity of the culture solution was measured. The minimum concentration when turbidity was not increased by the cultivation was used as the minimum inhibitory concentration (MIC). The results obtained are shown in Table 2 below. APZ, AP+Zn and AG+Zn were verified to have antibacterial action.

**Table 2**

| Sample | MIC (mg/ml) |
|---|---|
| APZ | 0.625 |
| AP+Zn | 1.25 |
| AG+Zn | 2.5 |
| APM | 10 |
| AG | 10 |

### Example 6:

### (Lipase Inhibitory Activity (1))

The activity of inhibiting lipase derived from *Pseudomonas* of the ascorbic acid-2-phosphate zinc salt, the ascorbic acid-2-phosphate and zinc salt mixture and the ascorbic acid-2-glucoside and zinc salt mixture of the present invention was examined.

*Pseudamonas*-derived lipase type XIII (produced by Sigma) was dissolved in 100 mM phosphate buffer (pH: 7.0) to have a final concentration of 43 mU/ml. 2 ml of the resulting solution was poured in a small test tube and thereto, APZ, AP+Zn and AG+Zn prepared as in Examples 1 to 3, APM and AG were added each to have concentrations of 1 mg/ml and 10 mg/ml. The reaction was started by addition of a substrate paranitrophenyl palmitate at a final concentration of 0.14 mg/ml. The reaction was performed at 30°C for 10 minutes under shaking. After completion of the reaction, the absorbance at a wavelength of 405 nm was measured and the difference from the absorbance of a control test tube where the substrate was not present was used as the activity. The ratio between activity when a sample was present and activity when the sample was not present was calculated as the inhibitory ratio of each sample and the values obtained were compared. The results are shown in Table 3 below. APZ, AP+Zn and AG+Zn were verified to have lipase inhibitory activity.

**Table 3**

| Sample | Inhibitory Ratio (%) | |
|---|---|---|
| | (1 mg/ml) | (10 mg/ml) |
| APZ | 76 | 98 |
| AP+Zn | 45 | 96 |
| AG+Zn | 43 | 92 |
| PM | 0 | 0 |
| AG | 0 | 0 |

### Example 7:

### (Lipase Inhibitory Activity (2))

The activity of inhibiting lipase derived from *Propionibacterium* of the ascorbic acid-2-phosphate zinc salt, the ascorbic acid-2-phosphate and zinc salt mixture and the ascorbic acid-2-glucoside and zinc salt mixture of the present invention was examined.

The culture solution of *Propionibacterium acnes* JCM6425 cultured in the same manner as in Example 7 was dialyzed with 50 mM phosphate buffer, then concentrated to about 1/10 the amount using an ultrafiltration film having a fraction molecular weight of 10,000 and used as the lipase partially purified product.

To 1.9 ml of 50 mM HEPES buffer (pH: 6.5) separately placed in a small test tube, 0.1 ml of the lipase partially purified product was added and thereto, APZ, AP+Zn and AG+Zn prepared in Examples 1 to 3, APM and AG were added each to have concentrations of 0.1 mg/ml, 1 mg/ml and 10 mg/ml. The reaction was started by addition of a substrate paranitrophenyl palmitate at a final concentration of 0.14 mg/ml. The reaction was performed at 30°C for 120 minutes under shaking. After completion of the reaction, the absorbance at a wavelength of 405 nm was measured and the difference from the absorbance of a control test tube where the substrate was not present was used as the activity. The ratio between activity when a sample was present and activity when the sample was not present was calculated as the inhibitory ratio of each sample and the values obtained were compared. The results are shown in Table 4 below. APZ, AP+Zn and AG+Zn were verified to have strong lipase inhibitory activity.

**Table 4**

| Sample | Inhibitory Ratio (%) | | |
|---|---|---|---|
| | (0.1 mg/ml) | (1 mg/ml) | (10 mg/ml) |
| APZ | 93 | 100 | 100 |
| AP+Zn | 80 | 99 | 100 |
| AG+Zn | 76 | 92 | 100 |
| APM | 0 | 0 | 0 |
| AG | 0 | 0 | 0 |

### Example 8:

### (Hyaluronidase Inhibitory Activity)

The activity of inhibiting hyaluronidase derived from *Propionibacterium* of the ascorbic acid-2-phosphate zinc salt, the ascorbic acid-2-phosphate and zinc salt mixture and the ascorbic acid-2-glucoside and zinc salt mixture of the present invention was examined.

The culture solution of *Propianibacterium acnes* JCM6425 cultured in the same manner as in Example 7 was dialyzed with 50 mM phosphate buffer, then concentrated to about 1/10 the amount using a membrane having a fraction molecular weight of 10,000 and used as the hyaluronidase partially purified product.

To 1.8 ml of 50 mM HEPES buffer (pH: 7.0) separately placed in a small test tube, 0.2 ml of the lipase partially purified product was added and thereto, APZ, AP+Zn and AG+Zn prepared in Examples 1 to 3, APM and AG were added each to have concentrations of 0.1 mg/ml, 1 mg/ml and 10 mg/ml. The reaction was started by addition of a substrate hyaluronic acid at a final concentration of 0.8 mg/ml. The reaction was performed at 30°C for 15 minutes under shaking. After the shaking, 0.1 ml of 0.2M potassium tetraborate was added, kept at 100°C for 3 minutes and cooled. To this solution, 3 ml of a paradimethylaminobenzaldehyde reagent (obtained by mixing and dissolving 10 g of dimethylaminobenzaldehyde, 12.5 ml of 10N hydrochloric acid and 77.5 ml of glacial acetic acid) 10 times diluted with glacial acetic acid was added and left standing at 37°C for 20 minutes to allow reaction with N-acetylglucosamine liberated to proceed and thereby cause coloring. The absorbance at a wavelength of 544 nm was measured and the difference from the absorbance of a control test tube where the substrate was not present was used as the activity. The ratio between activity when a sample was present and activity when the sample was not present was calculated as the inhibitory ratio of each sample and the values obtained were compared. The results are shown in Table 5 below. APZ, AP+Zn and AG+Zn were verified to have strong hyaluronidase inhibitory activity.

**Table 5**

| Sample | Inhibitory Ratio (%) | | |
|---|---|---|---|
| | (0.1 mg/ml) | (1 mg/ml) | (10 mg/ml) |
| APZ | 80 | 98 | 100 |
| AP+Zn | 65 | 99 | 96 |
| AG+Zn | 44 | 90 | 100 |
| APM | 22 | 32 | 34 |
| AG | 10 | 13 | 22 |

### Example 9:

### (Preparation of Dermal Agent)

Various dermal agents for preventing or treating comedones containing APZ, AP-Zn or AG-Zn prepared in Examples 1 to 3 were prepared as follows. Again, the blended amounts all are % by weight in the composition. The amounts with the balance to make a total amount of 100% are shown in Tables 6 and 7.

### (Lotion)

**Table 6**

| Standard Blended Ingredients Lotion 1-10 | Amount Blended |
|---|---|
| Sorbitol | 4.0 |
| Dipropylene glycol | 6.0 |
| PEG 1500 | 5.0 |
| POE(20) Oleyl alcohol | 0.5 |
| Methyl cellulose | 0.2 |
| Citric acid | 0.01 |
| Purified water | balance |
| Sodium hydroxide (adjusted to pH of 7.5) | trace |

**Table 7**

| Lotion | Specific Blended Ingredients and Amount Blended |
|---|---|
| Lotion 1 | APZ 0.3 |
| Lotion 2 | APZ 3.0 |
| Lotion 3 | AP+Zn 0.3 |
| Lotion 4 | AG+Zn 0.3 |
| Lotion 5 | APZ 0.3 APM 2.7 |
| Lotion 6 | AP+Zn 0.3, APM 2.7 |
| Lotion 7 | AP+Zn 0.3, APM 2.7 |

### (Blended Ingredients of Milky Lotion and Blended Amount Thereof)

The blended ingredients of milky lotion and the blended amounts thereof are shown in Table 8.

**Table 8**

| Ingredients Blended | Amount Blended |
|---|---|
| Glyceryl ether | 1.5 |
| Polyoxyethylene (20) hydrogenaged castor oil | 1.5 |
| Sorbitan monostearate | 1.0 |
| Squalane | 7.5 |
| Dipropylene glycol | 5.0 |
| Glaprizine | 0.2 |
| APZ | 0.3 |

### (Aqueous External Agents)

The blended ingredients of aqueous external agents (1) to (3) and the blended amount thereof are shown in Tables 9 to 11.

### (Aqueous External Agent (1))

**Table 9**

| Ingredients Blended | Amount Blended |
|---|---|
| Glyceryl monostearate | 1.0 |
| Isopropyl palmitate | 3.0 |
| Anhydrous lanolin | 1.0 |
| Glycerin | 5.0 |
| Methyl parahydroxybenzoate | 0.1 |
| Stearyl colaminoformyl pyridiumchloride | 1.5 |
| APZ | 3.0 |
| Glycyrrhiza nanking extract | 0.1 |
| Purified water | balance |

### (Aqueous External Agent (2))

**Table 10**

| Ingredients Blended | Amount Blended |
|---|---|
| Glyceryl monostearate | 1.0 |
| Isopropyl palmitate | 3.0 |
| Anhydrous lanolin | 1.0 |
| Glycerin | 5.0 |
| Methyl parahydroxybenzoate | 0.1 |
| Stearyl colaminoformyl pyridiumchloride | 1.5 |
| APZ | 1.0 |
| Tretinoin | 0.025 |
| Glycyrrhiza nanking extract | 0.1 |
| Purified water | balance |

### (Aqueous External Agent (3))

**Table 11**

| Ingredients Blended | Amount Blended |
|---|---|
| Glyceryl monostearate | 1.0 |
| Isopropyl palmitate | 3.0 |
| Anhydrous lanolin | 1.0 |
| Glycerin | 5.0 |
| Methyl parahydroxybenzoate | 0.1 |
| Stearyl colaminoformyl pyridiumchloride | 1.5 |
| AP+Zn | 1.0 |
| Tretinoin | 0.025 |
| Glycyrrhiza nanking extract | 0.1 |
| Purified water | balance |

Three kinds of aqueous external agent preparations were produced. More specifically, an aqueous external preparation containing AP+Zn and tretinoin (hereinafter simply referred to as "AP + Zn + tretinoin"), an aqueous external agent having the same composition as above except it contained only tretinoin but did not contain AP+Zn (hereinafter simply referred to as "tretinoin") and an aqueous external agent having the same composition as above except it contained only the base materials but did not contain tretinoin and AP+Zn (hereinafter simply referred to as "base materials") were produced. The preparations obtained were applied to 100 volunteers suffering from comedones to examine the effect on acne and measure the generation ratio of irritation such as rash or eczema.

The results are shown below. The effect on acne was rated 20 points when comedones were disappeared almost completely, 10 points when improvements were observed, and 0 point when no improvement was observed. The average thereof was obtained and the evaluation was excellent when the average grade was from 15 to 20 points, good when the average grade was from 5 to less than 15 points, and no change when the average grade was less than 5 points.

The generation ratio of irritation was obtained by a percentage of volunteers with irritation and the generation of irritation was evaluated as almost no irritation when the numerical value obtained was from 0 to less than 3%, as a slight irritation when the numerical value obtained was from 3 to less than 10%, as a medium irritation when the numerical value obtained was from 10 to less than 20%, and as irritated when the numerical value obtained was 20% or more. The results are shown in Table 12. (Evaluation of Effect on Treatment of Acne and Generation of Irritation)

**Table 12**

| Aqueous External Agent | Effect on Acne | Generation of Irritation |
|---|---|---|
| AP+Zn+tretinoin | Excellent | Slight irritation |
| Tretinoin | Excellent | Irritated |
| Base materials | No change | Almost no irritation |

### Example 10:

In 300 g of deionized water, 90 g of N-vinylacetamide and 10 g of sodium acrylate were dissolved. After purging the dissolved oxygen using nitrogen gas, the liquid temperature was adjusted to 20°C, 4 g of a 1% aqueous solution of 2,2'-azobis-2-amidinopropane dihydrochloride was added as a polymerization initiator, and the mixture was polymerized in a heat insulating state to obtain a hydrophilic polymer. The lump obtained was cut, dried and pulverized to obtain a powdered hydrophilic polymer.

### Formulation of Cataplasm

| | |
|---|---|
| Hydrophilic polymer | 6 parts by weight |
| Glycerin | 30 parts by weight |
| Dry aluminum hydroxide gel | 1 part by weight |
| Purified water | 62 parts by weight |
| Tartaric acid | 1 part by weight |
| Dermal agent | optimum |

The hydrophilic polymer prepared above and dry aluminum hydroxide gel were dispersed in glycerin and the resulting dispersion was gradually added to an aqueous tartaric acid solution and kneaded. Thereto, the dermal agent of the present invention was gradually added and kneaded.

The sol preparation obtained was spread on a polypropylene release film and then a non-woven fabric was applied onto the sol under pressure to obtain a cataplasm.

As proved in the Test Examples, the dermal agent comprising an ascorbic acid derivative which liberates in vivo ascorbic acid, and zinc salt, and the dermal agent comprising an ascorbic acid-2-phosphate zinc salt have antibacterial activity against *Propionibacterium*, antibacterial activity against *Staphylococcus*, lipase inhibitory activity and hyaluronidase inhibitory activity. Therefore, by virtue of the synergistic effect with other activities of the ascorbic acid derivative, these dermal agents are effective as a medicament for preventing or treating acne.

Furthermore, these medicaments have excellent safety and can be applied for a long period of time.

While the invention has been described in detail and with reference to specific embodiments thereof, it will be apparent to one skilled in the art that various changes and modifications can be made therein without departing from the spirit and scope thereof.

## Claims

1. A dermal agent for preventing or treating acne, (A) comprising a therapeutically effective amount of an ascorbic acid derivative which liberates ascorbic acid in vivo, or a salt thereof and a zinc salt compound or (B) comprising a therapeutically effective amount of a zinc salt of said ascorbic acid derivative.

2. An antibacterial dermal agent (A) comprising a therapeutically effective amount of an ascorbic acid derivative which liberates ascorbic acid in vivo, or a salt thereof and a zinc salt compound or (B) comprising a therapeutically effective amount of a zinc salt of said ascorbic acid derivative.

3. A dermal agent having inhibitory effect on growth of *Propionibacterium*, (A) comprising a therapeutically effective amount of an ascorbic acid derivative which liberates ascorbic acid in vivo, or a salt thereof and a zinc salt compound or (B) comprising a therapeutically effective amount of a zinc salt of said ascorbic acid derivative.

4. A dermal agent having inhibitory effect on growth of *Staphylococcus*, (A) comprising a therapeutically effective amount of an ascorbic acid derivative which liberates ascorbic acid in vivo, or a salt thereof and a zinc salt compound or (B) comprising a therapeutically effective amount of a zinc salt of said ascorbic acid derivative.

5. A dermal agent (A) comprising a therapeutically effective amount of an ascorbic acid derivative which liberates ascorbic acid in vivo, or a salt thereof and a zinc salt compound or (B) comprising a therapeutically effective amount of a zinc salt of said ascorbic acid derivative, said dermal agent having inhibitory activity against lipase derived from microorganisms.

6. A dermal agent (A) comprising a therapeutically effective amount of an ascorbic acid derivative which liberates ascorbic acid in vivo, or a salt thereof and a zinc salt compound or (B) comprising a therapeutically effective amount of a zinc salt of said ascorbic acid derivative, said dermal agent having inhibitory activity against hyaluronidase derived from microorganisms.

7. The dermal agent as claimed in any one of claims 1 to 6, wherein the ascorbic acid derivative which liberates ascorbic acid in vivo is a compound represented by the following formula (1): 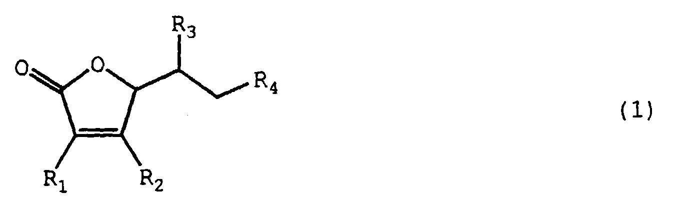 wherein R¹ and R² each represents a hydroxyl group, a phosphoric acid group, a pyrophosphoric acid group, a triphosphoric acid group, a polyphosphoric acid group, an O-glucosyl group, a sulfuric acid group, or an acyloxy group which may contain a branched or unsaturated bond, R³ and R⁴ each represents a hydroxyl group, a phosphoric acid group, a pyrophosphoric acid group, a triphosphoric acid group, a polyphosphoric acid group, an O-glucosyl group, a sulfuric acid group, an acyloxy group which may contain a branched or unsaturated bond, an alkyloxy group which may contain a branched or unsaturated bond, or a hydroxyalkyloxy group, and R³ and R⁴ may be bonded as an acetal or ketal to the same carbon atom through an oxygen atom, provided that R¹ and R² are not a hydroxyl group at the same time.

8. The dermal agent as claimed in any one of claims 1 to 6, wherein the salt of an ascorbic acid derivative which liberates ascorbic acid in vivo is a salt of ascorbic acid-2-phosphate represented by the following formula (2):

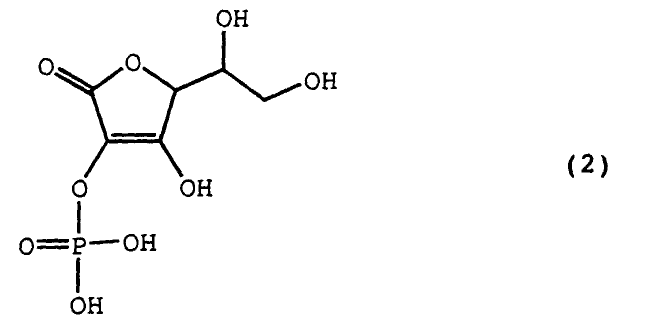

9. The dermal agent as claimed in any one of claims 1 to 6, wherein the zinc salt of an ascorbic acid derivative which liberates ascorbic acid in vivo is ascorbic acid-2-phosphate zinc salt represented by the following formula (3):

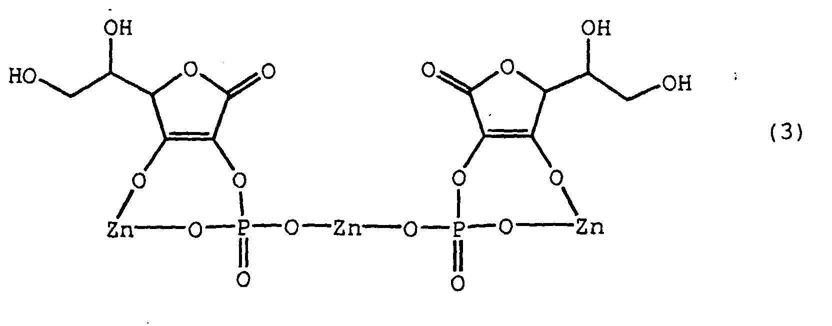

10. The dermal agent as claimed in any one of claims 1 to 6, wherein the ascorbic acid derivative which liberates ascorbic acid in vivo is ascorbic acid-2-O-glucoside.

11. A poultice comprising a hydrophilic resin and the dermal agent described in any one of claims 1 to 6 held therein.

12. The poultice as claimed in claim 11, wherein the hydrophilic resin is a polymer compound selected from the group consisting of acrylic acid polymers, N-vinylcarboxylic acid amide polymers, polyvinyl alcohols and acrylamide polymers.

13. The poultice as claimed in claim 12, wherein the N-vinylcarboxylic acid amide polymer is obtained by copolymerizing N-vinylacetamide and a copolymerizable compound having an ethylenic double bond in water.

14. A composition comprising tretinoin and an ascorbic acid derivative or a salt thereof, as described in any one of claims 1 to 6 in combination with tretinoin.

15. A method for relieving irritation of tretinoin, comprising applying to the skin the dermal agent described in any one of claims 1 to 6 in combination with tretinoin.
